# EUROPEAN PATENT SPECIFICATION

(11) **EP 2 523 687 B1**
(45) Date of publication and mention of the grant of the patent: **02.08.2017**
(21) Application number: 11701958.8
(22) Date of filing: 17.01.2011
(51) Int. Cl.: A61K 39/395, C07K 16/28, C12Q 1/68, G01N 33/00

(54) **Methods for diagnosis and treatment of cutaneous T cell lymphomas using the NKp46 receptor**
Auf NKp46-Rezeptor gerichtete Verfahren zur Diagnose und Behandlung von kutanen T-Zellen-Lymphomen
Procédés pour le diagnostic et le traitement de lymphomes cutanés à cellules T ciblant le récepteur NKp46

(30) Priority: 15.01.2010 EP 10305048
(43) Date of publication of application: 21.11.2012
(73) Proprietor: INSERM - Institut National de la Santé et de la Recherche Médicale, 75013 Paris (FR); Université Paris Diderot - Paris 7, 75205 Paris Cedex 13 (FR); Assistance Publique-Hôpitaux de Paris (AP-HP), 75004 Paris (FR)
(72) Inventor: BENSUSSAN, Armand, 75475 Cedex10 Paris (FR); MARIE-CARDINE, Anne, 75475 Cedex10 Paris (FR); BAGOT, Martine, 75475 Cedex10 Paris (FR)
(74) Representative: Cabinet Plasseraud
(86) International application number: PCT/EP2011/050524
(87) International publication number: WO 2011/086179

(56) References cited:
- WO-A2-2005/108415
- WO-A2-2006/103569
- WO-A2-2007/042573
- WO-A2-2007/071829
- BERTI E ET AL: "Natural killer and t-cell cytotoxic cutaneous lymphomas", MINERVA DERMATOLOGICA, EDIZIONI MINERVA MEDICA, TURIN, IT, vol. 140, no. 3, 1 January 2005 (2005-01-01), pages 249-261, XP009133897, ISSN: 0026-4741
- DULPHY NICOLAS ET AL: "NKG2D ligands expression and NKG2D-mediated NK activity in Sezary patients.", THE JOURNAL OF INVESTIGATIVE DERMATOLOGY FEB 2009 LNKD- PUBMED:18754036, vol. 129, no. 2, February 2009 (2009-02), pages 359-364, XP002583864, ISSN: 1523-1747

## Description

### FIELD OF THE INVENTION

The present invention relates to the diagnosis and treatment of Cutaneous T Cell Lymphomas (CTCL).

### BACKGROUND OF THE INVENTION

Cutaneous T Cell Lymphomas (CTCL) constitute a group of T lymphomas which primarily involve the skin. The CTCL group namely comprises mycosis fungoides, transformed mycosis fungoides, Sézary Syndrome, Lymphomatoide Papulosis and CD30+ cutaneous lymphomas. Sézary Syndrome is an aggressive form of CTCL characterized by a clonal expansion of CD4+/CD45RO+ T cells and the appearance of these malignant T cells in the blood.

The biology of CTCL disease, and particularly Sézary Syndrome, remains poorly understood. Current diagnosis of CTCL and particularly Sézary Syndrome is based on cytological and histological observations of the presence or absence of tumoral cells in a sample collected from the suspected body area (observation of histopathological aspect on skin biopsies and/or presence of Sézary Syndrome cells in the blood, via detection of cells showing a cerebriform nucleus). Such a diagnosis method is not fully reliable, notably at the early stages of the transformation of skin lymphocytes into malignant lymphocytes.

Today, CTCL therapy is tentatively achieved by induction of tumoral cell apoptosis via non specific chemotherapy.

New markers of circulating malignant Sézary cells (and other CTCL cells) have been recently described, especially CD158k/KIR3DL2 and SC5 (WO 2002 50122 patent application; Bagot M et al, 2001; Ortonne N et al, 2006; Poszepczynska-Guigne E et al, 2004; Bouaziz JD et al, 2009; Huet D et al, 2006). However, CD158k/KIR3DL2 expression appears in some patients to be heterogeneous into the tumour and about 10% of the patients lack it; likewise, SC5 appears to be also expressed on normal activated lymphocytes (Ortonne N et al, 2006). Thus, there is still a need for more accurate and appropriate solutions for the diagnosis and therapy of diseases involving the proliferation of malignant T cells, such as CTCL and more particularly Sézary Syndrome.

### SUMMARY OF THE INVENTION

The present invention relates to an *in vitro* method for diagnosing CTCL in a patient, said method comprising the step of assessing the expression of NKp46 receptor in T cells in a biological sample obtained from said patient, wherein the expression of NKp46 receptor in T cells is indicative of a CTCL.

Furthermore, the invention refers to an anti-NKp46 antibody for the treatment of CTCL and a pharmaceutical composition for the treatment of CTCL comprising an anti-NKp46 antibody of the invention.

Wherein said CTCL is the Sezary Syndrome.

### DETAILED DESCRIPTION OF THE INVENTION

The inventors showed that the NKp46, which is a specific NK cells receptor, is surprisingly expressed on peripheral blood malignant CD4+ T lymphocytes from patients with Sézary syndrome, an aggressive form of cutaneous T cell lymphoma. Thus, the NKp46 receptor may be used as a biomarker for the diagnosis and as a target for CTCL therapy.

### Definitions

Throughout the specification, several terms are employed and are defined in the following paragraphs.

The term "NKp46" (also called CD335, Ly94, MAR-1, NCR1 or RAR-1) has its general meaning in the art and refers to a cell surface receptor of NK cells identified by Sivori S et al (1997). The term may include naturally occurring NKp46 and variants and modified forms thereof. The NKp46 can be from any source, but typically is a mammalian (e.g., human and non-human primate) NKp46, particularly a human NKp46. An exemplary human native NKp46 amino acid sequence is provided in NP_004820.1 (GenPept database) and an exemplary human native nucleic acid sequence is provided in NM_004829.5 (GenBank database).

According to the present invention, "antibody" or "immunoglobulin" have the same meaning, and will be used equally in the present invention. The term "antibody" as used herein refers to immunoglobulin molecules and immunologically active portions of immunoglobulin molecules, i.e., molecules that contain an antigen binding site that immunospecifically binds an antigen. As such, the term antibody encompasses not only whole antibody molecules, but also antibody fragments as well as variants (including derivatives) of antibodies and antibody fragments. In natural antibodies, two heavy chains are linked to each other by disulfide bonds and each heavy chain is linked to a light chain by a disulfide bond. There are two types of light chain, lambda (1) and kappa (k). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from nonhypervariable or framework regions (FR) influence the overall domain structure and hence the combining site. Complementarity Determining Regions or CDRs refer to amino acid sequences which together define the binding affinity and specificity of the natural Fv region of a native immunoglobulin binding site. The light and heavy chains of an immunoglobulin each have three CDRs, designated L-CDR1, L-CDR2, L-CDR3 and H-CDR1, H-CDR2, H-CDR3, respectively. An antigen-binding site, therefore, includes six CDRs, comprising the CDR set from each of a heavy and a light chain V region. Framework Regions (FRs) refer to amino acid sequences interposed between CDRs.

The term "chimeric antibody" refers to an antibody which comprises a VH domain and a VL domain of an antibody of the invention, and a CH domain and a CL domain of a human antibody.

According to the invention, the term "humanized antibody" refers to an antibody having variable region framework and constant regions from a human antibody but retains the CDRs of the antibody of the invention.

The term "Fab" denotes an antibody fragment having a molecular weight of about 50,000 and antigen binding activity, in which about a half of the N-terminal side of H chain and the entire L chain, among fragments obtained by treating IgG with a protease, papaine, are bound together through a disulfide bond.

The term "F(ab')2" refers to an antibody fragment having a molecular weight of about 100,000 and antigen binding activity, which is slightly larger than the Fab bound via a disulfide bond of the hinge region, among fragments obtained by treating IgG with a protease, pepsin.

The term "Fab' " refers to an antibody fragment having a molecular weight of about 50,000 and antigen binding activity, which is obtained by cutting a disulfide bond of the hinge region of the F(ab')2.

A single chain Fv ("scFv") polypeptide is a covalently linked VH::VL heterodimer which is usually expressed from a gene fusion including VH and VL encoding genes linked by a peptide-encoding linker. "dsFv" is a VH::VL heterodimer stabilised by a disulfide bond. Divalent and multivalent antibody fragments can form either spontaneously by association of monovalent scFvs, or can be generated by coupling monovalent scFvs by a peptide linker, such as divalent sc(Fv)2.

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites.

The terms "biomarker" and "marker" are used herein interchangeably. They refer to a substance that is a distinctive indicator of a biological process, biological event, and/or pathologic condition.According to the invention, biological markers include surface antigens that are specifically expressed by cells from the immune system, particularly including by T lymphocytes.

As used herein, the term "patient" refers to a subject, preferably a human, that is suspected to be afflicted with a Cutaneous T Cell Lymphoma (CTCL). The term "biological sample" is used herein in its broadest sense. A biological sample is generally obtained from a patient. A sample may be of any biological tissue or fluid with which biomarkers of the present invention may be assayed. Frequently, a sample will be a "clinical sample", i.e., a sample derived from a patient. Such samples include, but are not limited to, bodily fluids which may or may not contain cells, e.g., blood (e.g., whole blood, serum or plasma). The term "biological sample" also encompasses any material derived by processing a biological sample. Derived materials include, but are not limited to, cells (or their progeny) isolated from the sample or proteins extracted from the sample. Processing of a biological sample may involve one or more of: filtration, distillation, extraction, concentration, inactivation of interfering components, addition of reagents, and the like.

In its broadest meaning, the term "treating" or "treatment" refers to reversing, alleviating, inhibiting the progress of the disorder or condition to which such term applies, or one or more symptoms of such disorder or condition.

### Diagnostic method of the invention

A first object of the invention relates to an *in vitro* method for diagnosing CTCL in a patient, said method comprising the step of assessing the expression of NKp46 receptor in T cells of a biological sample obtained from said patient, wherein the expression of NKp46 receptor in T cells is indicative of a CTCL.

In one embodiment of the invention, said *in vitro* method comprises the following steps of:
(i) detecting T cells in said biological sample,
(ii) detecting the expression of NKp46 in these T cells,
(iii) wherein the expression of NKp46 in these T cells is indicative of a CTCL.

The detection of T cells can be performed by detection of a specific T cell marker. Particularly, CD4 is used, but other marker can be used, such as CD3.

Thus, in one particular embodiment, the *in vitro* method of the invention comprises the following steps of:
(i) detecting the expression of CD4 in cells in said biological sample,
(ii) detecting the expression of NKp46 in cells in said biological sample,
(iii) wherein the expression of NKp46 and CD4 in these cells is indicative of a CTCL.

Said CTCL is the Sézary Syndrome.

According to the invention, biological sample that is used comprises T cells. In one embodiment of the invention, said biological sample is a whole blood sample.

Standard methods for detecting the expression of a specific surface marker such as NKp-46, CD3 or CD4 on cells are well known in the art. For example, the methods may consist in collecting a cell population from a patient and using differential binding partners directed against the specific surface markers (e.g. NKp-46, CD3 or CD4), wherein said cells are bound by said binding partners to said surface markers.

The binding with T cells markers such as CD4 (or CD3) permit to select T cells, and further binding with NKp46 permit to select the specific population of T cells expressing NKp46.

In a particular embodiment, the methods of the present disclosure comprise contacting the biological sample with a set of binding partners capable of selectively interacting with the specific surface markers such as NKp-46, CD3 or CD4. The binding partner may be an antibody that may be polyclonal or monoclonal, preferably monoclonal, directed against the specific surface markers. In another embodiment, the binding partners may be a set of aptamers.

Polyclonal antibodies of the disclosure or a fragment thereof can be raised according to known methods by administering the appropriate antigen or epitope to a host animal selected, e.g., from pigs, cows, horses, rabbits, goats, sheep, and mice, among others. Various adjuvants known in the art can be used to enhance antibody production. Although antibodies useful in practicing the invention can be polyclonal, monoclonal antibodies are preferred.

Monoclonal antibodies of the disclosure or a fragment thereof can be prepared and isolated using any technique that provides for the production of antibody molecules by continuous cell lines in culture. Techniques for production and isolation include but are not limited to the hybridoma technique; the human B-cell hybridoma technique; and the EBV-hybridoma technique.

In another embodiment, the binding partner may be an aptamer. Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by Exponential enrichment (SELEX) of a random sequence library, as described in Tuerk C. 1997. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consist of conformationally constrained antibody variable regions displayed by a platform protein, such as E. coli Thioredoxin A, that are selected from combinatorial libraries by two hybrid methods (Colas et al., 1996).

The binding partners such as antibodies or aptamers may be labelled with a detectable molecule or substance, such as a fluorescent molecule, a radioactive molecule or any others labels known in the art. Labels are known in the art that generally provide (either directly or indirectly) a signal.

As used herein, the term "labelled", with regard to the antibody or aptamer, is intended to encompass direct labelling of the antibody or aptamer by coupling (i.e., physically linking) a detectable substance, such as a radioactive agent or a fluorophore (e.g. fluorescein isothiocyanate (FITC) or phycoerythrin (PE) or Indocyanine (Cy5)) to the antibody or aptamer, as well as indirect labelling of the probe or antibody by reactivity with a detectable substance. An antibody or aptamer may be labelled with a radioactive molecule by any method known in the art. For example radioactive molecules include but are not limited radioactive atom for scintigraphic studies such as I123, I124, In111, Re186, Re188.

Preferably, the antibodies against the surface markers are already conjugated to a fluorophore (e.g. FITC-conjugated and/or PE-conjugated). Examples include monoclonal anti-human CD62E-FITC, CDC105-FITC, CD51-FITC, CD106-PE, CD31-PE, and CD54-PE, available through Ancell Co. (Bayport, Minn.).

The aforementioned assays may involve the binding of the binding partners (ie. Antibodies or aptamers) to a solid support. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like. The solid surfaces are preferably beads. Beads may be made of different materials, including but not limited to glass, plastic, polystyrene, and acrylic. In addition, the beads are preferably fluorescently labelled. In a preferred embodiment, fluorescent beads are those contained in TruCount(TM) tubes, available from Becton Dickinson Biosciences, (San Jose, California).

According to the invention, methods of flow cytometry are preferred methods for detecting the presence on or determining the level of cells expressing the specific surface markers such as NKp-46, CD3 or CD4 in the biological sample obtained from the patient.

Said methods are well known in the art (See e.g., (1976) Herzenber et al. (1976) Sci. Amer., 234:108) For example, fluorescence activated cell sorting (FACS) may be therefore used to separate in the biological sample the desired T cells (e.g. T cells expressing NKp46). In another embodiment, magnetic beads may be used to isolate endothelial microparticles (MACS).

For instance, beads labelled with monoclonal specific antibodies may be used for the positive selection of T cells expressing NKp46. For example, cells may be excited with 488 nm light and logarithmic green and red fluorescences of FITC and PE may be measured through 530/30 nm and 585/42 nm bandpass filters, respectively. The absolute number of T cells that express NKp46 may then be calculated through specific software useful in practicing the methods of the present invention.

Typically, a fluorescence activated cell sorting (FACS) method such as described in Example here below may be used to determining the levels of the desired T cells in the biological sample obtained from the patient.

Accordingly, in a specific embodiment, the method of the invention comprises the steps of obtaining a biological sample as above described; adding both labelled antibodies against surface markers that are specific to the desired T cells, putting said prepared sample into a container having a known number of solid surfaces wherein the solid surfaces are labelled with a fluorescent dye; performing a FACS flow cytometry on the prepared sample in order to calculate the absolute number of desired T cells therein.

Alternatively, the specific surface markers of T cells may be detected indirectly by measuring the expression of the genes encoding for such markers.

In a particular embodiment, the presence of mRNA of the cell surface markers can be determined both by in situ and by in vitro formats in a biological sample using methods known in the art. Many expression detection methods use isolated RNA.

The isolated mRNA can be used in hybridization or amplification assays that include, but are not limited to, Southern or Northern analyses, polymerase chain reaction (PCR) analyses and probe arrays. One preferred diagnostic method for the detection of mRNA levels involves contacting the isolated mRNA with a nucleic acid molecule (probe) that can hybridize to the mRNA encoded by the gene being detected. The nucleic acid probe can be, for example, a full-length cDNA, or a portion thereof, such as an oligonucleotide of at least 7, 15, 30, 50, 100, 250 or 500 nucleotides in length and sufficient to specifically hybridize under stringent conditions to a mRNA or genomic DNA encoding a marker of the present invention. Other suitable probes for use in the prognostic assays of the invention are described herein. Hybridization of an mRNA with the probe indicates that the marker in question is being expressed.

In one format, the mRNA is immobilized on a solid surface and contacted with a probe, for example by running the isolated mRNA on an agarose gel and transferring the mRNA from the gel to a membrane, such as nitrocellulose. In an alternative format, the probe(s) are immobilized on a solid surface and the mRNA is contacted with the probe(s), for example, in an Affymetrix gene chip array. A skilled artisan can readily adapt known mRNA detection methods for use in detecting the level of mRNA encoded by the markers of the present invention.

An alternative method for determining the presence of mRNA marker in a sample involves the process of nucleic acid amplification, e.g., by rtPCR, ligase chain reaction, self sustained sequence replication, transcriptional amplification system, Q-Beta Replicase, rolling circle replication or any other nucleic acid amplification method, followed by the detection of the amplified molecules using techniques well known to those of skill in the art.

### Kits

A further aspect of the disclosure provides kits comprising materials useful for carrying out diagnostic methods according to the present disclosure. The diagnosis procedures provided herein may be performed by diagnostics laboratories, experimental laboratories or practitioners. The disclosure provides kits that can be used in these different settings.

Material and reagents for detecting specific biomarkers of the disclosure in a biological sample for detecting CTCL in a subject may be assembled together in a kit.

Particularly, each kit comprises at least a binding partner of NKp46 and a binding partner of at least one T cell marker, such as CD4 (or CD3). Such binding partners are described above. More particularly, said binding partners are antibodies capable of detecting markers of interest.

Thus, a further object of the present disclosure relates to a kit for diagnosing CTCL in a patient, said kit comprising an antibody capable of detecting NKp46 and at least another antibody capable of detecting a T cell marker.

Particularly, said T cell marker is CD4. Thus, in a particular embodiment, in the kit, said antibody capable of detecting a T cell marker is an antibody capable of detecting CD4.

In one embodiment of the disclosure, said kit is used for diagnosing in a patient a CTCL selected from the group consisting of Mycosis Fungoides, transformed Mycosis Fungoides, Lymphomatoide Papulosis and CD30+ cutaneous lymphomas and Sézary Syndrome.

The binding partner can be tagged for an easier detection. It may or may not be immobilized on a substrate surface (e.g., beads, array, and the like). For example, an inventive kit may include an array for predicting the heart failure risk as provided herein. Alternatively, a substrate surface (e.g. membrane) may be included in an inventive kit for immobilization of the binding partner (e.g., via gel electrophoresis and transfer to membrane).

In addition, a kit of the disclosure generally also comprises at least one reagent for the detection of a complex between binding partner included in the kit and biomarker of the disclosure.

Depending on the procedure, the kit may further comprise one or more of: extraction buffer and/or reagents, western blotting buffer and/or reagents, and detection means. Protocols for using these buffers and reagents for performing different steps of the procedure may be included in the kit.

The different reagents included in a kit of the disclosure may be supplied in a solid (e.g. lyophilized) or liquid form. The kits of the present disclosure may optionally comprise different containers (e.g., vial, ampoule, test tube, flask or bottle) for each individual buffer and/or reagent. Each component will generally be suitable as aliquoted in its respective container or provided in a concentrated form. Other containers suitable for conducting certain steps of the disclosed methods may also be provided. The individual containers of the kit are preferably maintained in close confinement for commercial sale.

In certain embodiments, a kit comprises instructions for using its components for the CTCL diagnosis in a subject according to a method of the disclosure. Instructions for using the kit according to methods of the disclosure may comprise instructions for processing the biological sample obtained from the subject and/or for performing the test, or instructions for interpreting the results. A kit may also contain a notice in the form prescribed by a governmental agency regulating the manufacture, use or sale of pharmaceuticals or biological products.

### Methods of treatment of the invention

A further object of the invention relates to an anti-NKp46 antibody for use in the treatment of a CTCL.

Said CTCL is the Sézary Syndrome.

In one embodiment, the present invention relates to a method for treating a CTCL in a patient comprising the step of administrating said patient with an anti-NKp46 antibody.

According to the invention, an anti-NKp46 antibody is an antibody which binds to the NKp46 receptor and which is able to deplete or destroy cells expressing at their surface NKp46 in a patient.

Thus, the anti-NKp46 antibody is able to deplete malignant T cells expressing NKp46 (i.e. reduce circulating malignant T cell levels) in a patient treated therewith. Such depletion may be achieved via various mechanisms such antibody-dependent cell mediated cytotoxicity (ADCC) and/or complement dependent cytotoxicity (CDC), inhibition of malignant T cell proliferation and/or induction of malignant T cell death (e.g. via apoptosis).

"Complement dependent cytotoxicity" or "CDC" refers to the lysis of a target cell in the presence of complement. Activation of the classical complement pathway is initiated by the binding of the first component of the complement system to antibodies which are bound to their cognate antigen. To assess complement activation, a CDC assay, e.g. as described in Gazzano-Santoro et al. (1997) may be performed.

"Antibody-dependent cell-mediated cytotoxicity" or "ADCC" refers to a form of cytotoxicity in which secreted antibodies bound onto Fc receptors (FcRs) present on certain cytotoxic cells (e.g. Natural Killer (NK) cells, neutrophils, monocytes and macrophages) enable these cytotoxic effector cells to bind specifically to an antigen-bearing target cell and subsequently kill the target cell. To assess ADCC activity of a molecule of interest, an in vitro ADCC assay, such as that described in US Patent No. 5,500,362 or 5,821,337 may be performed.

Anti-NKp46 antibodies can be obtained according to conventional methods, such as hybridomas method as described above. Various adjuvants known in the art can be used to enhance antibody production. Although antibodies useful in practicing the invention can be polyclonal, monoclonal antibodies are preferred.

Then after raising antibodies as above described, the skilled man in the art can easily select those that deplete cells expressing NKp46, for example those that deplete cells via antibody-dependent cell mediated cytotoxicity (ADCC), complement dependent cytotoxicity (CDC), inhibition of cell proliferation or induction of cell death (e.g. via apoptosis).

Humanized antibodies and antibody fragments therefrom can also be prepared according to known techniques.

In a particular embodiment, the anti-NKp46 antibody is a chimeric, a humanized or a full-human anti-NKp46 antibody.

In another particular embodiment, the anti-NKp46 antibody is an antibody fragment selected form the group of F(ab')₂, F(ab)₂, Fab', Fab, Fv, scFv, and minimal recognition units.

The present disclosure also encompasses VHH, humanized VHH and VH antibodies.

Anti-NKp46 antibodies also include antibodies that destroy malignant T cells via other mechanisms. For example, these include radiolabelled antibodies that facilitate the destruction of malignant T cells by binding to the malignant T cell surface and delivering a lethal dose of radiation.

In a particular embodiment, the anti- NKp46 antibody may consist in an antibody conjugated to a cytotoxic agent or a growth inhibitory agent.

Accordingly, the invention contemplates the use of immunoconjugates comprising an anti- NKp46 antibody conjugated to a cytotoxic agent or a growth inhibitory agent.

A "growth inhibitory agent" when used herein refers to a compound or composition which inhibits growth of a cell, especially B cell, either in vitro or in vivo. Examples of growth inhibitory agents include agents that block cell cycle progression, such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, and 5-fluorouracil.

The term "cytotoxic agent" as used herein refers to a substance that inhibits or prevents the function of cells and/or causes destruction of cells. The term is intended to include radioactive isotopes (e.g. At211, I131, I125, Y90, Re186, Re188, Sm153, Bi212, P32, and radioactive isotopes of Lu), chemotherapeutic agents, e.g., methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin, including fragments and/or variants thereof, e.g., gelonin, ricin, saporin, and the various antitumor or anticancer agents disclosed below. Other cytotoxic agents are described below. A tumoricidal agent causes destruction of tumor cells.

Conjugation of the antibodies with cytotoxic agents or growth inhibitory agents may be made using a variety of bifunctional protein coupling agents including but not limited to N-succinimidyl (2-pyridyldithio) propionate (SPDP), succinimidyl (N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6diisocyanate), and bis-active fluorine compounds (such as 1,5-difluoro-2,4- dinitrobenzene). For example, a ricin immunotoxin can be prepared as described in Vitetta et al (1987). Carbon labeled 1-isothiocyanatobenzyl methyldiethylene triaminepentaacetic acid (MX-DTPA) is an exemplary chelating agent for conjugation of radionucleotide to the antibody (WO 94/11026).

Alternatively, a fusion protein comprising the antibody and cytotoxic agent or growth inhibitory agent may be made, by recombinant techniques or peptide synthesis. The length of DNA may comprise respective regions encoding the two portions of the conjugate either adjacent one another or separated by a region encoding a linker peptide which does not destroy the desired properties of the conjugate.

The anti-NKp46 antibody may be administered in the form of a pharmaceutical composition, as defined below.

Preferably, said anti-NKp46 antibody is administered in a therapeutically effective amount.

By a "therapeutically effective amount" is meant a sufficient amount of the Anti-NKp46 antibody to treat or to prevent atherosclerosis at a reasonable benefit/risk ratio applicable to any medical treatment.

It will be understood that the total periodically usage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidential with the specific polypeptide employed; and like factors well known in the medical arts. For example, it is well known within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the patient to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

### Pharmaceutical compositions

A further object of the invention relates to a pharmaceutical composition for use in the treatment of CTCL, said composition comprising an anti-NKp46 antibody. Said CTCL is the Sézary Syndrome.

The anti-NKp46 antibody may be combined with pharmaceutically acceptable excipients, and optionally sustained-release matrices, such as biodegradable polymers, to form therapeutic compositions.

In the pharmaceutical compositions of the present invention, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The anti-NKp46 antibody can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active polypeptides in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCI solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

The anti-NKp46 antibody may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

In addition to the compounds of the invention formulated for parenteral administration, such as intravenous or intramuscular injection, other pharmaceutically acceptable forms include, e.g. tablets or other solids for oral administration; liposomal formulations; time release capsules; and any other form currently used.

The invention will be further illustrated by the following figures and examples. However, these examples and figures should not be interpreted in any way as limiting the scope of the present invention.

### FIGURES

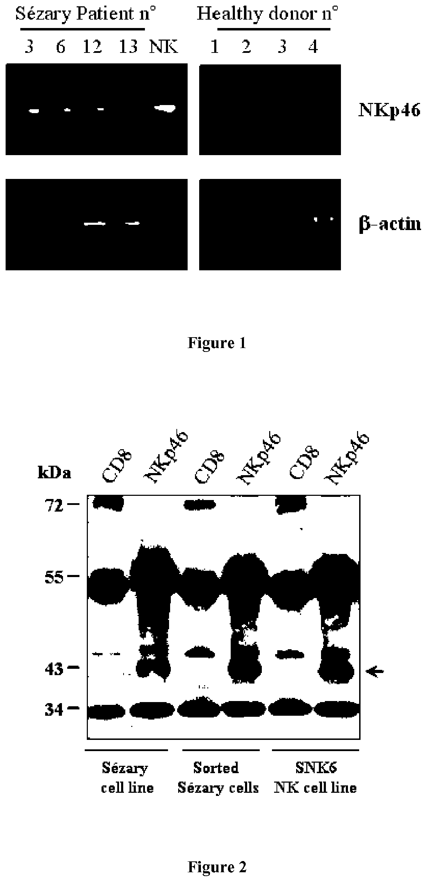

**Figure 1****.** RT-PCR was performed on sorted CD4⁺ T cells from patients 3, 6, 12 and 13 (see Table 1) (left panel) or healthy blood donors (right panel) using NKp46 or β-action specific primers. Total RNA extracted from purified peripheral blood NK cells of a healthy individual was used as positive control.
**Figure 2****.** Immunoprecipitates were prepared on cellular lysates of Pno Sézary cell line, SS patient sorted CD4⁺ T lymphocytes, or the NKp46⁺ NK cell line SNK6 using an anti-CD8 or -NKp46 mAb. After separation by reducing SDS-PAGE and transfer onto nitrocellulose, the precipitated proteins were revealed using an anti-NKp46 mAb followed by HRP-coupled secondary antibodies and an ECL detection system. The arrow indicates the position of NKp46 receptor.

### EXAMPLE 1: EXPRESSION AND FUNCTION OF THE NATURAL CYTOTOXICITY RECEPTOR NKP46 ON CIRCULATING MALIGNANT CD4 T LYMPHOCYTES OF SE'ZARY SYNDROME PATIENTS

### Material & Methods:

***Patients and cells.*** Fourteen patients were included in this study. For all patients, a diagnosis of Sézary syndrome (SS) was established based on clinical, histological and biologic criteria. As a control, blood from healthy volunteers was also collected. This study was approved by the institutional ethics committee (CCPPRB, Hopital Henri Mondor, Créteil, France). PBMCs were isolated by density gradient and CD4⁺ T lymphocytes further obtained by magnetic antibodies cell sorting (MACS) using anti-CD4-coupled beads according to the manufacturer's instructions (Miltenyi Biotec). NK cells were extracted from PBMCs by negative selection using a NK cell isolation kit (Miltenyi Biotec). The Sézary cell line Pno was generated in vitro (as described previously in Poszepczynska-Guigne E et al, 2004) and maintained in RPMI 1640 medium supplemented with 10% human serum (Institute of Biotechnologies, Lyon, France), glutamine, penicillin, streptomycin, IL2 (200U/ml; a gift from Sanofi-Avantis, Labège, France) and IL7 (5ng/ml; Peprotech). Where indicated, SS patients PBMCs were cultured overnight in the same conditions. The NK cell line SNK6 was grown in RPMI 1640 medium plus 10% SVF and IL2.

***FACS analysis.*** Triple immunolabelings were performed using anti-KIR3DL2/CD158k mAb (AZ158, IgG2a; kindly provided by Innate-Pharma, Marseille, France) plus FITC-conjugated goat-anti-mouse IgG secondary antibodies, PE-coupled anti-NKp46 mAb (Beckman Coulter, Villepinte, France) and PCy5-conjugated anti-CD3 mAb (Beckman Coulter). The corresponding control isotypic mAbs were used as negative controls. Analyses were realized using a FC500 cytometer (Beckman Coulter).

***RT***-***PCR***. Total RNAs were extracted using Trizol (Invitrogen) and subjected to reverse transcription as previously described (Namekawa T et al, 200). cDNA amplification was done according to standard procedures with specific primers for NKp46 leading to a product of 522 bp. Amplification of β-action was performed as internal control. PCR products were separated on a 1% agarose gel.

***Cell activation, immunoprecipitation and immunoblotting.*** Cells were incubated with 1 µg/ml of anti-CD3 (CD3x3, IgG1; produced locally) or NKp46 mAb (R&D Systems) alone or in combination for 5 min at RT. When necessary, anti-CD16 mAb was used to normalize the amount of antibodies added to the cells. Cell activation was conducted by addition of goat anti-mouse IgG antibodies (3 µg/ml; Jackson ImmunoResearch) and subsequent incubation for 5 min at 37°C. After washes and lysis, protein G Sepharose beads were directly added to post-nuclear supernatants in order to precipitate the receptors targeted during the activation step.

For NKp46 immunoprecipitation, anti-NKp46 mAb was added to cellular lysates and incubation conducted for 1h at 4°C. Anti-CD8 mAb (Beckman Coulter) was used as isotypic control mAb. Immunoprecipitates were subsequently collected with protein G Sepharose beads.

Immune complexes were separated by SDS-8% PAGE under reducing or nonreducing conditions, as indicated. Proteins were elecrotransferred onto a nitrocellulose membrane and subjected to immunoblotting with anti-phospho-CD3ζ (Tebu-Bio), -CD3ζ (Tebu-Bio), or -NKp46 (Tebu-Bio) mAbs. Revelation was realized with the appropriated HRP-conjugated secondary antibodies and an ECL detection system, according to the manufacturer's recommendations.

### Results

Our previous investigations regarding the expression of KIRs on PBMCs of patients with SS led to the characterization of KIR3DL2/CD158k as a reliable Sézary cell marker in most cases, and in rare occasions to the detection of additional HLA-C specific KIR at the malignant cell surface. These observations prompted us to further investigate the possible expression of the MHC class I-non-specific NK cell surface receptors NKp30, NKp44 and NKp46, that belong to the NCR family (Pessino A et al, 1998). Because our preliminary data did not give a clear interpretation regarding NKp30 and NKp44 expression profiles, we focused our attention on NKp46. Flow cytometry analyses allowed the detection of a CD158k⁺ population in all 14 patients with SS, accounting for 7.6 to 100% (mean: 51.1%) of the gated CD3⁺ T lymphocytes (Table 1). Note that these cells were identified as CD4⁺ T cells, excepted for patient 8 whom malignant cells lost expression of CD4 during the time course of the disease. Remarkably, a clear subset of CD3⁺NKp46⁺ cells was evidenced in PBMCs obtained from all but one (patient 9) SS patients (mean: 43.7%). Analysis of the mean % of the CD158k⁺NKp46⁺ cells revealed that this cell population displayed a CD3⁺ phenotype and therefore could not correspond to NK cells but to malignant Sézary cells (Table 1). Finally, a non significant percentage of CD3⁺CD158k⁺ or CD3⁺NKp46⁺ T lymphocytes was detected among the PBMCs isolated from healthy individuals (n=4, Table 1), confirming that this pattern of expression was specific of Sézary patient tumoral cells.

Although the expression of NCRs was shown to be unaffected in NK cells upon addition of cytokines, the expression of numerous T lymphocytes receptors was reported to be induced or increased under stimulating conditions. Flow cytometry analyses were therefore performed on cells that were exposed to a mixture of IL2 and IL7. Under these culture conditions, no major modification in the overall CD3⁺CD158k⁺NKp46⁺ T lymphocyte percentage was observed for all Sézary patients, with the exception of patient 9 (Table 1). Indeed, in this case, an increased percentage of CD3⁺CD158k⁺ T cells was obtained upon IL2+IL7 treatment. In addition NKp46 expression, that was not seen on freshly isolated cells, became detectable on the CD3⁺CD158k⁺ T cells following addition of interleukins, resulting in the detection of malignant cells exhibiting a CD3⁺CD158k⁺NKp46⁺ phenotype. In addition, while Sézary cells were found to express CD158k in most patients, a heterogeneous expression of CD158k by malignant T cell clones leading to the detection of a subset of CD158k⁻ tumoral cells was also evidenced (Ortonne N et al, 2006). In this latter case, the CD3⁺CD158k⁻ subpopulation was identified as NKp46⁺ cells, suggesting that a subset of malignant lymphocytes lacking CD158k expression might be efficiently detected through NKp46 immunolabeling upon exposure to interleukins. No interleukin-induced expression of NKp46 was seen on CD3⁺ T cells obtained from healthy donors (Table 1), inferring the unique expression profile observed for patients 6 and 9.

To ascertain the expression of NKp46 by Sézary cells, total RNA were prepared from sorted CD4⁺ T lymphocytes of SS patients or healthy individuals and used in RT-PCR experiments. As shown in Figure 1, the presence of NKp46 mRNA was evidenced in CD4⁺ T cells isolated from SS patients 3, 6, 12 and 13, but not in the one obtained from healthy blood donors. Double strand sequencing further confirmed that the amplified product corresponded to NKp46 cDNA. Note that the level of detected NKp46 mRNA was proportional to the percentage of CD3⁺NKp46⁺ circulating cells, a weak malignant population leading to a hardly detectable NKp46 transcript as for patient 13, while an elevated tumoral content corresponded to a strong detection of NKp46 mRNA (patients 3, 6 and 12). The expression of the NCR was further confirmed by anti-NKp46 immunoblotting performed on NKp46 immunoprecipitates prepared from cellular lysates of an in vitro generated Sézary cell line or patient sorted CD4⁺ T cells (Figure 2). Thus a specific and effective detection of NKp46 in both the Sézary cell line and circulating malignant cells from the representative patient 3 was observed, to an amount equivalent to the one obtained in a control NK cell line (Figure 2B). Altogether these data established that NKp46 is efficiently expressed in Sézary cells and might represent a new reliable marker of the circulating malignant cells.

**Table 1: Expression of CD158k and NKp46 by Sézary patients PBMC**

| **% CD3⁺CD158k⁺** | | **% CD3⁺NKp46⁺** | | **% CD158k⁺NKp46⁺** | | |
|---|---|---|---|---|---|---|
| | **Medium*^{a}*** | **+IL2+IL7*^{a}*** | **Medium** | **+IL2+IL7** | **Medium** | **+IL2+IL7** |
| Patient 1 | 38.6 | 44.3 | 8 | 37 | 8.4 | 26.6 |
| Patient 2 | 100 | 100 | 100 | 100 | 100 | 100 |
| Patient 3 | 91.2 | 80.3 | 90.5 | 90.8 | 94.1 | 86.7 |
| Patient 4 | 16.3 | 15.4 | 15.7 | 13 | 11.2 | 11.9 |
| Patient 5 | 9.8 | 9.1 | 9.9 | 11.2 | 10.6 | 9.1 |
| Patient 6 | 85.5 | 71.8 | 44.3 | 94.7 | 44.6 | 59.6 |
| Patient 7 | 7.6 | 8.6 | 5.6 | 6.5 | 4.9 | 4.5 |
| Patient 8 | 100 | 100 | 100 | 100 | 100 | 100 |
| Patient 9 | 50.3 | 100 | 0 | 100 | 0 | 100 |
| Patient 10 | 15.6 | 15.8 | 15.9 | 21.3 | 17 | 21.3 |
| Patient 11 | 9.2 | 11.2 | 8.6 | 10.6 | 7.8 | 9.6 |
| Patient 12 | 100 | 100 | 100 | 100 | 100 | 100 |
| Patient 13 | 12.8 | 15.8 | 13.3 | 14.5 | 5.1 | 14.5 |
| Patient 14 | 98.3 | 98.6 | 100 | 100 | 100 | 100 |
| **Mean** | **52.5** | **55.1** | **50.2** | **65.4** | **48.7** | **60.6** |
| | | | | | | |
| Healthy A | <0.2 | nd | <0.2 | <0.2 | nd | nd |
| Healthy B | <0.2 | nd | <0.2 | <0.2 | nd | nd |
| Healthy C | <0.2 | nd | <0.2 | <0.2 | nd | nd |
| Healthy D | <0.2 | nd | <0.2 | <0.2 | nd | nd |
| **Mean** | **<0.2** | **nd** | **<0.2** | **<0.2** | **nd** | **nd** |

| | | | | | | |
|---|---|---|---|---|---|---|
| *^{a}* Freshly isolated PBMC of Sezary syndrome patients or healthy donors were cultured overnight with culture media alone or supplemented with IL-2 and IL-7 as described in Materials and Methods | | | | | | |

### EXAMPLE 2: EXPRESSION OF NKp46 BY SKIN LYMPHOCYTES ISOLATED FROM CD30+ CUTANEOUS LYMPHOMAS AND MYCOSIS FUNGOIDES BIOPSIES.

Skin biopsies from patients with CD30+ cutaneous lymphoma and Mycosis Fungoides were obtained. To determine whether other types of CTCLs expressed NKp46, we isolated lymphocytes from skin biopsies obtained from patients with CD30+ cutaneous lymphoma or Mycosis Fungoides (Oshtory S, Apisarnthanarax N, Gilliam AC, Cooper KD, Meyerson HJ.Usefulness of flow cytometry in the diagnosis of mycosis fungoides. J Am Acad Dermatol. 2007;57:454-62.). Flow cytometry analysis reveals the presence of a significant subset of CD3+NKp46+ cells in gated lymphocytes. These results clearly established that NKp46 is a specific marker for all types of CTCLs including with CD30+ cutaneous lymphoma and Mycosis Fungoides. Importantly, the fact that non transformed Mycosis Fungoides expressed NKp46 indicates that it could correspond to an early marker for CTCLs diagnosis. As shown figure 1 for Sézary Syndrome, similar detection of NKp46 mRNA was obtained with skin lymphocytes isolated from Mycosis Fungoides biopsies.

### REFERENCES

Throughout this application, various references describe the state of the art to which this invention pertains.
Bagot M, Moretta A, Sivori S, et al. CD4(+) cutaneous T-cell lymphoma cells express the p140-killer cell immunoglobulin-like receptor. Blood. 2001;97:1388-1391.
Bouaziz JD, Remtoula N, Bensussan A, Marie-Cardine A, Bagot M. Absolute CD3+ CD158k+ lymphocyte count is reliable and more sensitive than cytomorphology to evaluate blood tumour burden in Sezary syndrome. Br J Dermatol. 2009.
Huet D, Bagot M, Loyaux D, Capdevielle J, Conraux L, Ferrara P, Bensussan A*, Marie-Cardine A. SC5 mAb represents a unique tool for the detection of extracellular vimentin as a specific marker of Sezary cells. J Immunol, 2006, 176(1):652-9
Ortonne N, Huet D, Gaudez C, et al. Significance of circulating T-cell clones in Sezary syndrome. Blood. 2006;107:4030-4038.
Namekawa T, Snyder MR, Yen JH, et al. Killer cell activating receptors function as costimulatory molecules on CD4+CD28null T cells clonally expanded in rheumatoid arthritis. J Immunol. 2000;165:1138-1145.
Pessino A, Sivori S, Bottino C, et al. Molecular cloning of NKp46: a novel member of the immunoglobulin superfamily involved in triggering of natural cytotoxicity. J Exp Med. 1998;188:953-960.
Poszepczynska-Guigne E, Schiavon V, D'Incan M, et al. CD158k/KIR3DL2 is a new phenotypic marker of Sezary cells: relevance for the diagnosis and follow-up of Sezary syndrome. J Invest Dermatol. 2004;122:820-823.
Sivori S, Vitale M, Morelli L, Sanseverino L, Augugliaro R, Bottino C, Moretta L, Moretta A. p46, a novel natural killer cell-specific surface molecule that mediates cell activation. J Exp Med. 1997 Oct 6;186(7):1129-36.

## Claims

1. An *in vitro* method for diagnosing a cutaneous T cell lymphoma (CTCL) which is the Sézary Syndrome in a patient, said method comprising the step of assessing the expression of NKp46 receptor in T cells in a biological sample obtained from said patient, wherein the expression of NKp46 receptor in T cells is indicative of the CTCL.

2. The *in vitro* method of claim 1, said method comprising the following steps of:
(i) detecting T cells in said biological sample,
(ii) detecting the expression of NKp46 in these T cells,
(iii) wherein the expression of NKp46 receptor in these T cells is indicative of the CTCL.

3. The *in vitro* method of claim 2, said method comprising the following steps of:
(i) detecting the expression of CD4 in cells in said biological sample,
(ii) detecting the expression of NKp46 in cells in said biological sample,
(iii) wherein the expression of NKp46 and CD4 in these cells is indicative of the CTCL.

4. The *in vitro* method according to anyone of claims 1-3 wherein said biological is whole blood sample.

5. An anti-NKp46 antibody for use in the treatment of a cutaneous T cell lymphoma (CTCL) which is the Sézary Syndrome.

6. The anti-NKp46 antibody for use according to claim 5 wherein said antibody is a chimeric or humanized anti-NKp46 antibody.

7. The anti-NKp46 antibody for use according to anyone of claims 5-6 wherein said antibody is an antibody conjugated to a cytotoxic agent or a growth inhibitory agent.

8. A pharmaceutical composition for use in the treatment of a CTCL which is the Sézary Syndrome said composition comprising an anti-NKp46 antibody according to anyone of claims 5-7.

## Patentansprüche

1. Ein in-vitro-Verfahren zur Diagnose eines kutanen T-Zell-Lymphoms (CTCL), welches das Sézary-Syndrom bei einem Patienten ist, wobei das Verfahren den Schritt der Bestimmung der Expression des NKp46-Reptors in T-Zellen in einer biologischen Probe, die aus diesem Patienten erhalten wurde, umfasst, wobei die Expression des NKp46-Rezeptors in T-Zellen CTCL indiziert.

2. Das in-vitro-Verfahren nach Anspruch 1, wobei das Verfahren die folgenden Schritte umfasst:
(i) Detektion von T-Zellen in dieser biologischen Probe,
(ii) Detektion der Expression von NKp46 in diesen T-Zellen,
(iii) wobei die Expression des NKp46-Rezeptors in diesen T-Zellen CTCL indiziert.

3. Das in-vitro-Verfahren nach Anspruch 2, wobei das Verfahren die folgenden Schritte umfasst:
(i) Detektion der Expression von CD4 in Zellen in dieser biologischen Probe,
(ii) Detektion der Expression von NKp46 in Zellen in dieser biologischen Probe,
(iii) wobei die Expression von NKp46 und CD4 in diesen Zellen CTCL indiziert.

4. Das in-vitro-Verfahren nach einem der Ansprüche 1 bis 3, wobei diese biologische eine Vollblutprobe ist.

5. Ein Anti-NKp46-Antikörper zur Verwendung bei der Behandlung eines kutanen T-Zell-Lymphoms (CTCL), welches das Sézary-Syndrom ist.

6. Der Anti-NKp46-Antikörper zur Verwendung nach Anspruch 5, wobei dieser Antikörper ein chimärer oder humanisierter Anti-NKp46-Antikörper ist.

7. Der Anti-NKp46 Antikörper zur Verwendung nach einem der Ansprüche 5 bis 6, wobei dieser Antikörper ein Antikörper ist, der an ein zytotoxisches Agens oder an ein wachstumsinhibierendes Agens konjugiert ist.

8. Eine pharmazeutische Zusammensetzung zur Verwendung bei der Behandlung von CTCL, welches das Sézary-Syndrom ist, wobei diese Zusammensetzung einen Anti-NKp46-Antikörper nach einem der Ansprüche 5 bis 7 umfasst.

## Revendications

1. Procédé *in vitro* de diagnostic du lymphome T cutané (LTC) qui est le syndrome de Sézary chez un patient, ledit procédé comprenant l'étape d'estimation de l'expression du récepteur NKp46 dans les lymphocytes T dans un échantillon biologique obtenu dudit patient, dans lequel l'expression du récepteur NKp46 dans les lymphocytes T indique le LTC.

2. Procédé *in vitro* selon la revendication 1, ledit procédé comprenant les étapes suivantes :
(i) la détection des lymphocytes T dans ledit échantillon biologique,
(ii) la détection de l'expression du NKp46 dans ces lymphocytes T,
(iii) dans lequel l'expression du récepteur NKp46 dans ces lymphocytes T indique le LTC.

3. Procédé *in vitro* selon la revendication 2, ledit procédé comprenant les étapes suivantes :
(i) la détection de l'expression du CD4 dans des cellules dans ledit échantillon biologique,
(ii) la détection de l'expression du NKp46 dans des cellules dans ledit échantillon biologique,
(iii) dans lequel l'expression du NKp46 et du CD4 dans ces cellules indique le LTC.

4. Procédé *in vitro* selon l'une quelconque des revendications 1 à 3, dans lequel ledit échantillon biologique est un échantillon de sang total.

5. Anticorps anti-NKp46 pour une utilisation dans le traitement d'un lymphome T cutané (LTC) qui est le syndrome de Sézary.

6. Anticorps anti-NKp46 pour une utilisation selon la revendication 5, où ledit anticorps est un anticorps anti-NKp46 chimérique ou humanisé.

7. Anticorps anti-NKp46 pour une utilisation selon l'une quelconque des revendications 5 ou 6, dans lequel ledit anticorps est un anticorps conjugué à un agent cytotoxique ou à un agent inhibiteur de croissance.

8. Composition pharmaceutique pour une utilisation dans l'e traitement d'un LTC qui est le syndrome de Sézary, ladite composition comprenant un anticorps anti-NKp46 selon l'une quelconque des revendications 5 à 7.
